# EUROPÄISCHE PATENTSCHRIFT

(11) **EP 1 918 022 B1**
(45) Veröffentlichungstag und Bekanntmachung des Hinweises auf die Patenterteilung: **18.11.2015**
(21) Anmeldenummer: 07018199.5
(22) Anmeldetag: 17.09.2007
(51) Int. Cl.: B01J 20/20, C01B 31/08

(54) **Hochleistungsadsorbentien auf Basis von Aktivkohle mit hoher Mikroporosität**
High performance adsorbents based on active carbon with high micro-porosity
Adsorbants à haute capacité à base de charbon actif ayant une microporosité élevée

(30) Priorität: 12.10.2006 DE 102006048790
(43) Veröffentlichungstag der Anmeldung: 07.05.2008
(73) Patentinhaber: Blücher GmbH, 40699 Erkrath (DE)
(72) Erfinder: von Blücher, Hasso, 40699 Erkrath (DE); Böhringer, Bertram, Dr., 42115 Wuppertal (DE); Giebelhausen, Jann-Michael, 14712 Rathenow (DE)
(74) Vertreter: Von Rohr Patentanwälte Partnerschaft mbB

(56) Entgegenhaltungen:
- WO-A-02/32569
- DE-A1- 19 930 732
- US-A- 5 576 261
- US-A- 5 993 766
- THIEME RÖMPP ONLINE: "Akivkohle" August 2004 (2004-08), GEORG THIEME VERLAG , XP002468792 Gefunden im Internet: URL:http://www.roempp.com/prod/index1.html > [gefunden am 2008-02-13] * Zusammenfassung *

## Beschreibung

Die vorliegende Erfindung betrifft das technische Gebiet der Adsorption. Insbesondere betrifft die vorliegende Erfindung Hochleistungsadsorbentien auf der Basis von Aktivkohle mit hoher Mikroporosität sowie deren Verwendung, insbesondere für Adsorptionsfiltermaterialien, für die Adsorption von Giftstoffen, Schadstoffen und Gerüchen, insbesondere aus Gas- bzw. Luftströmen, zur Reinigung oder Aufbereitung von Gasen, wie insbesondere Luft, zur Anwendung in der Medizin bzw. Pharmazie, als Sorptionsspeicher für Gase, insbesondere Wasserstoff, und dergleichen.

Aktivkohle ist aufgrund ihrer recht unspezifischen adsorptiven Eigenschaften das am meisten angewendete Adsorbens. Gesetzliche Auflagen, aber auch das steigende Bewußtsein der Verantwortung für die Umwelt, führen zu einem steigenden Bedarf an Aktivkohle.

Aktivkohle wird im allgemeinen durch Carbonisierung (synonym auch als Schwelung, Pyrolyse, Abbrand etc. bezeichnet) und anschließende Aktivierung kohlenstoffhaltiger Verbindungen erhalten, wobei solche Verbindungen bevorzugt werden, die zu ökonomisch vernünftigen Ausbeuten führen. Denn die Gewichtsverluste durch Abspalten flüchtiger Bestandteile beim Carbonisierung und durch den Abbrand beim Aktivieren sind erheblich. Für weitere Einzelheiten der Aktivkohleherstellung kann beispielsweise verwiesen werden auf H. v. Kienle und E. Bäder, Aktivkohle und ihre industrielle Anwendung, Enke Verlag Stuttgart, 1980.

Die Beschaffenheit der erzeugten Aktivkohle - fein- oder grobporig, fest oder brüchig etc. - hängt vom Ausgangsmaterial ab. Übliche Ausgangsmaterialien sind Kokosnußschalen, Holzabfälle, Torf, Steinkohle, Peche, aber auch besondere Kunststoffe, die unter anderem bei der Herstellung von Aktivkohlegeweben eine gewisse Rolle spielen.

Aktivkohle wird in verschiedenen Formen verwendet: Pulverkohle, Splitterkohle, Kornkohle, Formkohle und seit Ende der siebziger Jahre auch kugelförmige Aktivkohle ("Kugelkohle"). Kugelförmige Aktivkohle hat gegenüber anderen Formen von Aktivkohle wie Pulver-, Splitter-, Kornkohle und dergleichen eine Reihe von Vorteilen, die sie für bestimmte Applikationen wertvoll oder sogar unverzichtbar macht: Sie ist rieselfähig, abriebfest bzw. staubfrei und hart. Kugelkohle ist wegen ihrer speziellen Form, aber auch wegen der hohen Abriebfestigkeit beispielsweise für besondere Einsatzgebiete sehr gefragt.

Kugelkohle wird heute noch meist durch mehrstufige und sehr aufwendige Verfahren hergestellt. Das bekannteste Verfahren besteht in der Herstellung von Kügelchen aus Steinkohlenteerpech und geeigneten asphaltartigen Rückständen der Erdölchemie, welche oxidiert werden - damit sie unschmelzbar werden -, und geschwelt und aktiviert werden. Beispielsweise kann die Kugelkohle auch in einem mehrstufigen Verfahren ausgehend von Bitumen hergestellt werden. Diese mehrstufigen Verfahren sind sehr kostenintensiv, und der damit verbundene hohe Preis dieser Kugelkohle verhindert viele Anwendungen, bei denen die Kugelkohle aufgrund ihrer Eigenschaften eigentlich bevorzugt werden müßte.

In der WO 98/07655 A1 wird ein Verfahren zur Herstellung von Aktivkohlekügelchen beschrieben, bei dem zunächst eine Mischung, die einen aus der Diisocyanatherstellung stammenden Destillationsrückstand, einen kohlenstoffhaltigen Verarbeitungshilfsstoff und gegebenenfalls einen oder mehrere weitere Zusatzstoffe umfaßt, zu rieselförmigen Kügelchen verarbeitet wird und anschließend die auf diese Weise erhaltenen Kügelchen carbonisiert und dann aktiviert werden.

Aus dem Stand der Technik bekannt ist ferner die Herstellung von Kugelkohle durch Schwelung und anschließende Aktivierung von neuen oder gebrauchten Ionenaustauschern, die Sulfonsäuregruppen enthalten, bzw. durch Schwelung von Ionenaustauschervorstufen in Gegenwart von Schwefelsäure und anschließende Aktivierung, wobei die Sulfonsäuregruppen bzw. die Schwefelsäure die Funktion eines Vernetzers haben. Solche Verfahren sind beispielsweise in der DE 43 28 219 A1 und in der DE 43 04 026 A1 sowie in der DE 196 00 237 A1 einschließlich der deutschen Zusatzanmeldung DE 196 25 069 A1 beschrieben. Weiterhin wird in der DE 199 30 732 A1 ein Verfahren zur gesteuerten Herstellung von Kugelaktivkohle, insbesondere mittels Carbonisierung und gegebenenfalls nachfolgender Aktivierung von sulfonierten Styrol/Divinylbenzol-Copolymeren, beschrieben.

Bei speziellen Anwendungen sind aber nicht nur die Geometrie bzw. die äußere Gestalt der Aktivkohle von entscheidender Bedeutung, sondern auch deren Porosität, insbesondere das Gesamtporenvolumen und die Adsorptionskapazität einerseits und die Verteilung der Poren, d. h. der Anteil an Mikro-, Meso- und Makroporen in bezug auf das Gesamtporenvolumen, andererseits.

Bei einer Reihe von Anwendungen ist eine besonders hohe Mikroporosität der Aktivkohle, d. h. ein großer Mikroporenvolumenanteil, bei insgesamt hohem Gesamtporenvolumen gefragt, so beispielsweise bei den eingangs genannten Anwendungen, so z. B. für die Adsorption von Giftstoffen, Schadstoffen und Gerüchen, insbesondere aus Gas- bzw. Luftströmen, zur Reinigung oder Aufbereitung von Gasen, wie insbesondere Luft, zur Anwendung in der Medizin bzw. Pharmazie, bei der sorptiven Speicherung von Gasen, insbesondere Wasserstoff, bei der Herstellung von Adsorptionsfiltermaterialien (z. B. für ABC-Schutzbekleidung) und dergleichen.

Die aus dem Stand der Technik zu diesem Zweck bekannte Aktivkohle weißt zwar eine gewisse Mikroporosität auf, die aber nicht in allen Fällen ausreichend ist. Zudem beobachtet man mit zunehmender Porosität oftmals eine unerwünschte, bisweilen inakzeptable Abnahme der mechanischen Stabilität bzw. Abriebfestigkeit. Auch sind der Anteil an Mikroporen am Gesamtporenvolumen und das absolute Mikroporenvolumen nicht immer ausreichend, um für alle Anwendungen eine ausreichende Leistungsfähigkeit zu gewährleisten.

Es ist daher eine Aufgabe der vorliegenden Erfindung, ein insbesondere für die vorgenannten Anwendungsgebiete geeignetes Hochleistungsadsorbens auf der Basis von Aktivkohle bereitzustellen, welches die zuvor geschilderten Nachteile des Standes der Technik zumindest weitgehend vermeidet oder aber wenigstens abschwächt. Insbesondere sollte das erfindungsgemäß bereitzustellende Adsorbens eine hohe Mikroporosität, d. h. einen hohen Mikroporenanteil in bezug auf das Gesamtporenvolumen sowie ein großes Mikroporenvolumen, aufweisen, dennoch gleichzeitig eine gute mechanische Beständigkeit, insbesondere eine hohe Abrieb- und Berstbeständigkeit, aufweisen.

Zur Lösung des zuvor geschilderten Problems schlägt die vorliegende Erfindung - gemäß einem **ersten** Aspekt der vorliegenden Erfindung - Hochleistungsadsorbentien auf der Basis von Aktivkohle in Form von diskreten Aktivkohlekörnern, vorzugsweise in Kugelform, nach Anspruch 1 vor. Weitere, insbesondere vorteilhafte Ausgestaltungen der erfindungsgemäßen Hochleistungsadsorbentien sind Gegenstand der diesbezüglichen Unteransprüche. Weiterer Gegenstand der vorliegenden Erfindung ist - gemäß einem **zweiten** Aspekt der vorliegenden Erfindung - die erfindungsgemäße Verwendung der Hochleistungsadsorbentien nach der vorliegenden Erfindung, wie sie in den diesbezüglichen Verwendungsansprüchen näher definiert ist. Gegenstand der vorliegenden Erfindung sind somit - gemäß einem **ersten** Aspekt der vorliegenden Erfindung - Hochleistungsadsorbentien auf der Basis von Aktivkohle in Kugelform mit mittleren Teilchendurchmessern, bestimmt nach ASTM D2862-97/04, im Bereich von 0,01 bis 1,0 mm, welche die folgenden Parameter aufweisen:
- ein Gesamtporenvolumen nach Gurvich von mindestens 0,7 cm³/g, wobei mindestens 70 % dieses Gesamtporenvolumens durch Mikroporen mit Porendurchmessern von ≤ 2 nm (20 Å) gebildet sind,
- einen mittleren Porendurchmesser von höchstens 3 nm (30 Å), bestimmt auf Basis der Stickstoffisothermen
- eine BET-Oberfläche von mindestens 1.500 m²/g bestimmt nach ASTM D6556-04 und
- eine Schüttdichte, bestimmt nach ASTM B527-93/00, im Bereich von 350 bis 600 g/l.

Die erfindungsgemäßen Hochleistungsadsorbentien bzw. Aktivkohlen zeichnen sich insbesondere durch eine große Gesamtporosität und eine gleichzeitig große BET-Oberfläche aus. Wie nachfolgend noch ausgeführt wird, ist trotz der hohen Porosität auch die mechanische Belastbarkeit, insbesondere die Abriebfestigkeit und die Berst- bzw. Druckbelastbarkeit, der erfindungsgemäßen Hochleistungsadsorbentien - im Unterschied zu vergleichbaren hochporösen Aktivkohlen des Standes der Technik - extrem hoch, so daß die erfindungsgemäßen Hochleistungsadsorbentien bzw. Aktivkohlen auch für solche Anwendungen geeignet sind, bei denen sie großen mechanischen Belastungen ausgesetzt sind.

Bei allen vorstehend genannten und noch im folgenden genannten Parameterangaben ist zu beachten, daß die aufgeführten Grenzwerte, insbesondere Ober- und Untergrenzen, mitumfaßt sind, d. h. alle Werteangaben verstehen sich einschließlich der jeweiligen Grenzen. Weiterhin versteht es sich von selbst, daß es einzelfallbedingt oder anwendungsbezogen gegebenenfalls erforderlich sein kann, geringfügig von den genannten Grenzwerten abzuweichen, ohne daß der Rahmen der vorliegenden Erfindung verlassen ist.

Die vorgenannten und im folgenden noch genannten Parameterangaben werden mit genormten oder explizit angegebenen Bestimmungsverfahren oder dem Fachmann an sich geläufigen Bestimmungsmethoden bestimmt. Die Parameterangaben betreffend die Charakterisierung der Porosität ergeben sich jeweils aus der Stickstoffisotherme der vermessenen Aktivkohle.

Was die Bestimmung des Gesamtporenvolumens nach Gurvich anbelangt, so handelt es sich um eine dem Fachmann auf diesem Gebiet an sich bekannte Meß-/Bestimmungsmethode. Zu weitergehenden Einzelheiten bezüglich der Bestimmung des Gesamtporenvolumens nach Gurvich kann beispielsweise verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Die Bestimmung der spezifischen Oberfläche gemäß BET ist dem Fachmann grundsätzlich als solches bekannt, so daß diesbezüglich keine weitergehenden Einzelheiten ausgeführt werden zu brauchen. Alle BET-Oberflächenangaben beziehen sich auf die Bestimmung gemäß ASTM D6556-04. Im Rahmen der vorliegenden Erfindung wird zur Bestimmung der BET-Oberfläche die sogenannte MultiPoint-BET-Bestimmungsmethode (MP-BET) in einem Partialdruckbereich p/p₀ von 0,05 bis 0,1 angewendet.

In bezug auf weitergehende Einzelheiten zur Bestimmung der BET-Oberfläche bzw. zu der BET-Methode kann auf die vorgenannte ASTM D6556-04 sowie auf Römpp Chemielexikon, 10. Auflage, Georg Thieme Verlag, Stuttgart/New York, Stichwort: "BET-Methode", einschließlich der dort referierten Literatur und auf Winnacker-Küchler (3. Auflage), Band 7, Seiten 93 ff. sowie auf Z. Anal. Chem. 238, Seiten 187 bis 193 (1968) verwiesen werden.

Die Bestimmung des mittleren Porendurchmessers erfolgt auf Basis der jeweiligen Stickstoffisothermen.

Das Gesamtporenvolumen nach Gurvich der erfindungsgemäßen Hochleistungsadsorbentien beträgt mindestens 0,7 cm³/g, insbesondere mindestens 0,8 cm³/g, vorzugsweise mindestens 0,9 cm³/g, besonders bevorzugt mindestens 1,0 cm³/g, und kann Werte bis zu 1,5 cm³/g, insbesondere bis zu 1,6 cm³/g, vorzugsweise bis zu 1,8 cm³/g, erreichen.

Im allgemeinen liegt das Gesamtporenvolumen nach Gurvich der erfindungsgemäßen Hochleistungsadsorbentien im Bereich von 0,7 bis 1,8 cm³/g, insbesondere 0,8 bis 1,6 cm³/g, vorzugsweise 0,9 bis 1,5 cm³/g.

Eine Besonderheit der erfindungsgemäßen Hochleistungsadsorbentien ist unter anderem darin zu sehen, daß sie über ein sehr großes Gesamtporenvolumen nach Gurvich verfügen, so daß eine große Adsorptionskapazität bereitgestellt wird, wobei ein hoher Anteil auf Mikroporen entfällt.

Im allgemeinen sind mindestens 70 %, insbesondere mindestens 75 %, vorzugsweise mindestens 80 %, besonders bevorzugt mindestens 85 %, ganz besonders bevorzugt mindestens 90 %, des Gesamtporenvolumens nach Gurvich der erfindungsgemäßen Hochleistungsadsorbentien durch Mikroporen mit Porendurchmessern von ≤ 20 Å gebildet.

Im allgemeinen ist 70 % bis 95 %, insbesondere 75 bis 90 %, vorzugsweise 75 bis 85 %, des Gesamtporenvolumens nach Gurvich der erfindungsgemäßen Hochleistungsadsorbentien durch Mikroporen mit Porendurchmessern von ≤ 20 Å gebildet.

Im Rahmen der vorliegenden Erfindung bezeichnet der Begriff der Mikroporen solche Poren mit Porendurchmessern bis zu 20 Å einschließlich, wohingegen der Begriff der Mesoporen solche Poren mit Porendurchmessern von > 20 Å bis 50 Å einschließlich bezeichnet und der Begriff der Makroporen solche Poren mit Porendurchmessern > 50 Å bezeichnet.

Aufgrund ihrer hohen Mikroporosität ist das Mikroporenvolumen der erfindungsgemäßen Hochleistungsadsorbentien relativ hoch: Im allgemeinen liegt das durch Mikroporen mit Porendurchmessern von ≤ 20 Å gebildete Mikroporenvolumen nach Carbon Black der erfindungsgemäßen Hochleistungsadsorbentien im Bereich von 0,5 bis 1,4 cm³/g, insbesondere 0,6 bis 1,2 cm³/g, bevorzugt 0,7 bis 1,1 cm³/g.

Die Bestimmungsmethode nach Carbon Black ist dem Fachmann an sich bekannt, so daß es diesbezüglich keiner weitergehenden Einzelheiten bedarf. Zudem kann zu weitergehenden Einzelheiten der Bestimmung der Porenoberfläche und des Porenvolumens nach Carbon Black beispielsweise verwiesen werden auf R. W. Magee, Evaluation of the External Surface Area of Carbon Black by Nitrogen Adsorption, Presented at the Meeting of the Rubber Division of the American Chem. Soc., Oktober 1994, z. B. referiert in: Quantachrome Instruments, AUTOSORB-1, AS1 WinVersion 1.50, Operating Manual, OM, 05061, Quantachrome Instruments 2004, Florida, USA, Seiten 71 ff.

Aufgrund der hohen Mikroporosität der erfindungsgemäßen Hochleistungsadsorbentien ist der mittlere Porendurchmesser relativ gering: Im allgemeinen beträgt er höchstens 30 Å, insbesondere höchstens 26 Å, vorzugsweise höchstens 25 Å, ganz besonders bevorzugt höchstens 24 Å. Im allgemeinen liegt der mittlere Porendurchmesser der erfindungsgemäßen Hochleistungsadsorbentien im Bereich von 15 bis 30 Å, insbesondere 16 bis 26 Å, vorzugsweise 17 bis 25 Å, besonders bevorzugt 18 bis 24 Å.

Wie zuvor ausgeführt, ist eine Besonderheit der erfindungsgemäßen Hochleistungsadsorbentien in der relativ großen BET-Oberfläche zu sehen, welche mindestens 1.500 m²/g, vorzugsweise mindestens 1.525 m²/g, besonders bevorzugt mindestens 1.550 m²/g, ganz besonders bevorzugt mindestens 1.575 m²/g, beträgt.

Im allgemeinen liegt die BET-Oberfläche der erfindungsgemäßen Hochleistungsadsorbentien im Bereich von 1.500 m²/g bis 2.750 m²/g, insbesondere 1.525 bis 2.500 m²/g, vorzugsweise 1.550 bis 2.400 m²/g, besonders bevorzugt 1.575 bis 2.350 m²/g.

Auch das gewichts- und volumenbezogene Volumen V_{ads} (N₂) der erfindungsgemäßen Hochleistungsadsorbentien bei unterschiedlichen Partialdrücken p/p₀ ist sehr groß:
So beträgt das gewichtsbezogene adsorbierte N₂-Volumen V_{ads (gew)} der erfindungsgemäßen Hochleistungsadsorbentien, bestimmt bei einem Partialdruck p/p₀ von 0,25, mindestens 400 cm³/g, insbesondere mindestens 420 cm³/g, und liegt insbesondere im Bereich von 400 bis 800 cm³/g, vorzugsweise 410 bis 750 cm³/g, besonders bevorzugt 420 bis 700 cm³/g.

Im allgemeinen beträgt das volumenbezogene adsorbierte N₂-Volumen V_{ads (vol.)} der erfindungsgemäßen Hochleistungsadsorbentien, bestimmt bei einem Partialdruck p/p₀ von 0,25, mindestens 200 cm³/cm³, insbesondere mindestens 220 cm³/cm³, und liegt insbesondere im Bereich von 200 bis 300 cm³/cm³, vorzugsweise 210 bis 275 cm³/cm³, besonders bevorzugt 225 bis 260 cm³/cm³.

Im allgemeinen beträgt das gewichtsbezogene adsorbierte N₂-Volumen V_{ads (gew.)} der erfindungsgemäßen Hochleistungsadsorbentien, bestimmt bei einem Partialdruck p/p₀ von 0,995, mindestens 450 cm³/g, insbesondere mindestens 460 cm³/g, und liegt insbesondere im Bereich von 450 bis 900 cm³/g, vorzugsweise 460 bis 875 cm³/g, besonders bevorzugt 470 bis 850 cm³/g.

Im allgemeinen beträgt das volumenbezogene adsorbierte N₂-Volumen V_{ads (vol.)} der erfindungsgemäßen Hochleistungsadsorbentien, bestimmt bei einem Partialdruck p/p₀ von 0,995, mindestens 250 cm³/cm³, insbesondere mindestens 260 cm³/cm³, und liegt insbesondere im Bereich von 250 bis 400 cm³/cm³, vorzugsweise 260 bis 350 cm³/cm³, besonders bevorzugt 265 bis 320 cm³/cm³.

Eine weitere Besonderheit der erfindungsgemäßen Hochleistungsadsorbentien ist die große Mikroporenoberfläche, d. h. die große Oberfläche, welche durch Poren mit Porendurchmesser von ≤ 20 Å gebildet wird. Im allgemeinen beträgt die aus Poren mit Porendurchmessern von ≤ 20 Å gebildete Mikorporenoberfläche nach Carbon Black der erfindungsgemäßen Hochleistungsadsorbentien mindestens 1.400 m²/g, insbesondere mindestens 1.450 m²/g, vorzugsweise mindestens 1.500 m²/g, und liegt im allgemeinen im Bereich von 1.400 bis 2.500 m²/g, insbesondere 1.450 bis 2.400 m²/g, vorzugsweise 1.500 bis 2.300 m²/g.

Darüber hinaus verfügen die erfindungsgemäßen Hochleistungsadsorbentien über eine extrem hohe Butanadsorption und gleichzeitig eine extrem hohe Iodzahl, was ihre Eigenschaft charakterisiert, exzellente Adsorptionseigenschaften in bezug auf verschiedenste zu adsorbierende Stoffe aufzuweisen.

So liegt die gemäß ASTM D5742-95/00 bestimmte Butanadsorption der erfindungsgemäßen Hochleistungsadsorbentien im allgemeinen bei mindestens 25 %, insbesondere bei mindestens 30 %, vorzugsweise bei mindestens 40 %. Im allgemeinen weisen die erfindungsgemäßen Hochleistungsadsorbentien eine nach ASTM D5742-95/00 bestimmte Butanadsorption im Bereich von 25 % bis 80 %, insbesondere 30 bis 70 %, vorzugsweise 35 bis 65 %, auf.

Die nach ASTM D4607-94/99 bestimmte Iodzahl der erfindungsgemäßen Hochleistungsadsorbentien liegt im allgemeinen bei mindestens 1.350 mg/g, insbesondere bei mindestens 1.450 mg/g, bevorzugt bei mindestens 1.500 mg/g. Bevorzugterweise weisen die erfindungsgemäßen Hochleistungsadsorbentien eine nach ASTM D4607-94/99 bestimmte Iodzahl im Bereich von 1.350 bis 2.100 mg/g, insbesondere 1.450 bis 2.050 mg/g, vorzugsweise 1.500 bis 2.000 mg/g, auf.

Trotz der hohen Porosität, insbesondere Mikroporosität, weisen die erfindungsgemäßen Hochleistungsadsorbentien eine hohe Druck- bzw. Berstfestigkeit (Gewichtsbelastbarkeit) sowie eine extrem hohe Abriebfestigkeit auf.

So liegt die Druck- bzw. Berstfestigkeit (Gewichtsbelastbarkeit) pro Aktivkohlekom, insbesondere pro Aktivkohlekügelchen, bei mindestens 10 Newton, insbesondere mindestens 15 Newton, vorzugsweise mindestens 20 Newton. Im allgemeinen variiert die Druck- bzw. Berstfestigkeit (Gewichtsbelastbarkeit) pro Aktivkohlekorn, insbesondere pro Aktivkohlekügelchen, im Bereich von 10 bis 50 Newton, insbesondere 12 bis 45 Newton, vorzugsweise 15 bis 40 Newton.

Wie zuvor ausgeführt, ist auch die Abriebshärte der erfindungsgemäßen Hochleistungsadsorbentien extrem hoch: So liegt die Abriebfestigkeit nach der Methode gemäß CEFIC (Conseil Europeen des Federations des l'Industrie Chimique, Avenue Louise 250, Bte 71, B - 1050 Brüssel, November 1986, European Council of Chemical Manufacturers' Federations, Testmethoden für Aktivkohlen, Ziffer 1.6 "Mechanische Härte", Seiten 18/19) stets bei 100 %. Auch nach ASTM D3802 werden Abriebfestigkeiten der erfindungsgemäßen Hochleistungsadsorbentien von stets 100 % erhalten.

Daher hat die Anmelderin eine modifizierte Testmethode in Anlehnung an diese CEFIC-Methode entwickelt, um aussagekräftigere Werte zu erhalten. Die modifizierte Bestimmungsmethode simuliert besser den Widerstand der Probe bzw. der Hochleistungsadsorbentien gegenüber Abrieb oder Zerreiben unter praxisnahen Bedingungen. Zu diesem Zweck wird die Probe eine definierte Zeit lang in einem horizontal schwingenden, mit einer Wolframcarbidkugel beschickten Mahlbecher unter genormten Bedingungen beansprucht. Zu diesem Zweck wird wie folgt vorgegangen: 200 g einer Probe werden eine Stunde lang bei (120 ± 2) °C im Umlufttrockenschrank (Typ: Heraeus UT 6060 der Fa. Kendro GmbH, Hanau) getrocknet und anschließend in einem Exsikkator über Trockenmittel auf Raumtemperatur abgekühlt. 50 g der getrockneten Probe werden entnommen und mittels einer Siebmaschine mit Analysensieb (Typ: AS 200 control der Fa. Retsch GmbH, Hanau) bei einer Schwinghöhe von 1,2 mm zehn Minuten lang über ein Analysensieb (Analysensieb der Maschenweite: 0,315 mm, Durchmesser: 200 mm, Höhe: 50 mm) abgesiebt; das Unterkorn wird verworfen. 5 ml des Nennkornes werden in einen 10-ml-Meßzylinder nach DIN ISO 384 (Volumen: 10 ml, Höhe: 90 mm) abgefüllt und das Gewicht mittels eines Wägeglases mit eingeschliffenem Glasdeckel (Volumen: 15 ml, Durchmesser: 35 mm, Höhe: 30 mm) auf 0,1 mg genau bestimmt mittels Analysenwaage (Typ: BP121S der Sartorius AG, Göttingen, Wägebereich: 120 g, Genauigkeitsklasse: E2, Ablesbarkeit: 0,1 mg). Die abgewogene Probe wird zusammen mit einer Wolframcarbidmahlkugel mit 20 mm Durchmesser in einen 25-ml-Mahlbecher mit Schraubverschluß (Volumen: 25 ml, Durchmesser: 30 mm, Länge: 65 mm, Werkstoff: Edelstahl) gegeben und dann der Abriebtest mittels Schwingmühle (Typ: MM301 der Fa. Retsch GmbH, Haan, Schwingmühle mit Mahlbecher) durchgeführt; dabei schwingt der Mahlbecher in horizontaler Lage eine Minute lang mit einer Frequenz von 10 Hz in der Schwingmühle, was dazu führt, daß die Mahlkugel auf die Probe einschlägt und somit Abrieb erzeugt. Anschließend wird die Probe mittels einer Siebmaschine bei einer Schwinghöhe von 1,2 mm fünf Minuten lang über das vorgenannte Analysensieb abgesiebt, wobei das Unterkorn wieder verworfen und das Nennkorn größer 0,315 mm auf 0,1 mg genau im Wägeglas mit Deckel zurückgewogen wird. Die Berechnung der Abriebhärte erfolgt als Masseanteil in % nach folgender Formel: Abriebhärte [%] = (100 x Rückwaage [g]) / Einwaage [g].

Gemäß dieser von der Anmelderin modifizierten Bestimmungsmethode in Abwandlung der vorgenannten CEFIC-Norm beträgt die Abriebfestigkeit der erfindungsgemäßen Hochleistungsadsorbentien mindestens 95 %, insbesondere mindestens 96 %, vorzugsweise mindestens 97 %, besonders bevorzugt mindestens 98 %, ganz besonders bevorzugt mindestens 99 %.

Die erfindungsgemäßen Hochleistungsadsorbentien sind auf Basis kugelförmiger Aktivkohle ausgebildet, deren mittlerer Teilchendurchmesser, bestimmt nach ASTM D2862-97/04, im Bereich von 0,01 bis 1,0 mm, , insbesondere 0,1 bis 0,8 mm, vorzugsweise 0,2 bis 0,7 mm, besonders bevorzugt 0,4 bis 0,55 mm, variiert.

Der Aschegehalt der erfindungsgemäßen Hochleistungsadsorbentien, bestimmt nach ASTM D2866-94/04, beträgt höchstens 1 %, insbesondere höchstens 0,8 %, vorzugsweise höchstens 0,6 %, besonders bevorzugt höchstens 0,5%.

Der nach ASTM D2867-04/04 bestimmte Feuchtigkeitsgehalt der erfindungsgemäßen Hochleistungsadsorbentien beträgt höchstens 1 %, insbesondere höchstens 0,5 %, vorzugsweise höchstens 0,2 %.

Die erfindungsgemäßen Hochleistungsadsorbentien weisen erfindungsgemäß eine Schüttdichte (Bulk Density), bestimmt nach ASTM B527-93/00, im Bereich von 350 bis 600 g/l auf.

Was das äußere Porenvolumen nach Carbon Black der erfindungsgemäßen Hochleistungsadsorbentien anbelangt, so liegt dieses im allgemeinen im Bereich von 0,05 bis 0,5 cm³/g, insbesondere 0,1 bis 0,45 cm³/g. Im allgemeinen bildet das äußere Porenvolumen nach Carbon Black der erfindungsgemäßen Hochleistungsadsorbentien höchstens 35 %, vorzugsweise höchstens 30 %, des Gesamtporenvolumens, insbesondere 10 % bis 35 %, bevorzugt 14 bis 30 %, des Gesamtporenvolumens.

Was die äußere Porenoberfläche nach Carbon Black der erfindungsgemäßen Hochleistungsadsorbentien anbelangt, so liegt diese im allgemeinen im Bereich von 50 bis 300 m²/g, insbesondere 60 bis 250 m²/g, bevorzugt 70 bis 200 m²/g. Im allgemeinen bildet die äußere Porenoberfläche nach Carbon Black der erfindungsgemäßen Hochleistungsadsorbentien höchstens 15 %, vorzugsweise höchstens 10 %, der Gesamtporenoberfläche, insbesondere 4 % bis 15 %, bevorzugt 4 bis 12 %, der Gesamtporenoberfläche.

Was die Herstellung der erfindungsgemäßen Hochleistungsadsorbentien anbelangt, so sind diese durch Carbonisierung und nachfolgende Aktivierung von gelförmigen sulfonierten Styrol/Divinylbenzol-Copolymeren, insbesondere sulfonierten divinylbenzolvernetzten Polystyrolen, in Kornform, bevorzugt in Kugelform, erhältlich. Der Divinylbenzolgehalt der als Ausgangsmaterialien zur Herstellung der erfindungsgemäßen Hochleistungsadsorbentien verwendeten sulfonierten Styrol/Divinylbenzol-Copolymeren sollte insbesondere im Bereich von 1 bis 15 Gew.-%, vorzugsweise 2 bis 10 Gew.-%, bezogen auf die Styrol/Divinylbenzol-Copolymere, liegen. Die Ausgangscopolymere müssen vom Geltyp ausgewählt sein, damit sich eine mikroporöse Struktur ausbilden kann.

Wenn von unsulfonierten Ausgangsmaterialien ausgegangen wird, kann die Sulfonierung in situ durchgeführt werden, insbesondere mit dem Fachmann an sich bekannten Methoden, vorzugsweise mittels Schwefelsäure und/oder Oleum. Dies ist dem Fachmann an sich geläufig.

Als Ausgangsmaterialien besonders bewährt haben sich gelförmigen Typen von den entsprechenden Ionenaustauscherharzen bzw. den entsprechenden Vorstufen von Ionenaustauscherharzen, welche noch sulfoniert werden müssen.

Bei der Carbonisierung (synonym auch als Pyrolyse, Abbrand oder Schwelung bezeichnet) erfolgt die Umwandlung der kohlenstoffhaltigen Ausgangspolymere zu Kohlenstoff, d. h. mit anderen Worten wird das kohlenstoffhaltige Ausgangsmaterial verkohlt. Bei der Carbonisierung der zuvor genannten, gelförmigen organischen Polymerkörnern, insbesondere Polymerkügelchen, auf Basis von Styrol und Divinylbenzol, die Sulfonsäuregruppen enthalten, führt die Abspaltung der Sulfonsäuregruppen während der Carbonisierung zu freien Radikalen und somit zu Vernetzungen, ohne die es keinen Pyrolyserückstand (= Kohlenstoff) gäbe. Im allgemeinen wird die Carbonisierung unter inerter Atmosphäre (z. B. Stickstoff) oder allenfalls leicht oxidierender Atmosphäre durchgeführt. Gleichermaßen kann es vorteilhaft sein, während der Carbonisierung, insbesondere bei höheren Temperaturen (z. B. im Bereich von etwa 500 bis 650 °C), zu der Inertatmosphäre eine kleinere Menge an Sauerstoff, insbesondere in Form von Luft (z. B. 1 bis 5 %), zuzugeben, um eine Oxidation des carbonisierten Polymerskeletts zu bewirken und auf diese Weise die nachfolgende Aktivierung zu erleichtern. Im allgemeinen wird die Carbonisierung bei Temperaturen von 100 bis 950 °C, insbesondere 150 bis 900 °C, vorzugsweise 300 bis 850 °C, durchgeführt. Die Gesamtdauer der Carbonisierung liegt dabei etwa bei 30 Minuten bis 6 Stunden.

Im Anschluß an die Carbonisierung wird das carbonisierte Zwischenprodukt einer Aktivierung unterzogen, am Ende derer die erfindungsgemäßen Hochleistungsadsorbentien auf Basis von Aktivkohle in Kornform, insbesondere Kugelform, resultieren. Das Grundprinzip der Aktivierung besteht darin, einen Teil des bei der Carbonisierung generierten Kohlenstoffs selektiv und gezielt unter geeigneten Bedingungen abzubauen. Hierdurch entstehen zahlreiche Poren, Spalten und Risse, und die auf die Masseneinheit bezogene Oberfläche nimmt erheblich zu. Bei der Aktivierung wird also ein gezielter Abbrand der Kohle vorgenommen. Da bei der Aktivierung Kohlenstoff abgebaut wird, tritt bei diesem Vorgang ein Substanzverlust ein, welcher - unter optimalen Bedingungen - gleichbedeutend mit einer Erhöhung der Porosität und Zunahme der inneren Oberfläche und des Porenvolumens ist. Die Aktivierung erfolgt daher unter selektiven bzw. kontrollierten oxidierenden Bedingungen. Die Aktivierung wird im allgemeinen bei Temperaturen von 700 bis 1.300 °C, insbesondere 800 bis 1.200 °C, vorzugsweise 900 bis 1.100 °C, durchgeführt.

Die Besonderheit bei der Herstellung der erfindungsgemäßen Hochleistungsadsorbentien ist - neben der Auswahl des zuvor beschriebenen Ausgangsmaterials - in der speziellen Verfahrensführung der Aktivierung zu sehen, insbesondere in der Dauer der Aktivierung in Kombination mit der ausgewählten Aktivierungsatmosphäre. Die Anmelderin hat überraschenderweise herausgefunden, daß - wenn man die Aktivierung extrem lange betreibt, insbesondere 12 bis 30 Stunden lang, vorzugsweise 19 bis 23 Stunden lang, und dabei eine nur schwach oxidierende Atmosphäre, welche nur geringe Mengen an Wasserdampf in einer ansonsten stickstoffhaltigen Atmosphäre enthält, verwendet, und zwar in Mengen von nur etwa 0,1 bis 5 Vol.-%, insbesondere 0,5 bis 4 Vol.-%, Wasserdampf - ausgehend von den ausgewählten Ausgangsmaterialien die erfindungsgemäßen Hochleistungsadsorbentien hoher Mikroporosität und hoher mechanischer Stabilität mit den übrigen zuvor geschilderten Eigenschaften resultieren.

Insbesondere ist überraschend, daß zum einen die extrem lange Aktivierungsdauer nicht zu einem schädlichen, übermäßigen Abbrand unter erheblichem Substanzverlust führt und zum anderen trotz der hohen Porosität bei gleichzeitig hoher Mikroporosität eine extrem hohe Abriebfestigkeit und mechanische Druckfestigkeit resultiert. Es war nicht zu erwarten, daß derart lange Aktivierungsdauern nicht zu einem nachteiligen Ergebnis führen würden und daß bei derart langen Aktivierungsdauern überwiegend selektiv die Mikroporosität bzw. das Mikroporenvolumen generiert wird, sofern von den gelförmigen Ausgangsmaterialien, wie zuvor definiert, ausgegangen wird.

Durch Variation der Aktivierungsdauern, insbesondere im Bereich von 12 bis 30 Stunden, vorzugsweise 19 bis 23 Stunden, kann dann gezielt die Mikroporosität eingestellt werden. Auf diese Weise können die erfindungsgemäßen Hochleistungsadsorbentien sozusagen maßgeschneidert werden.

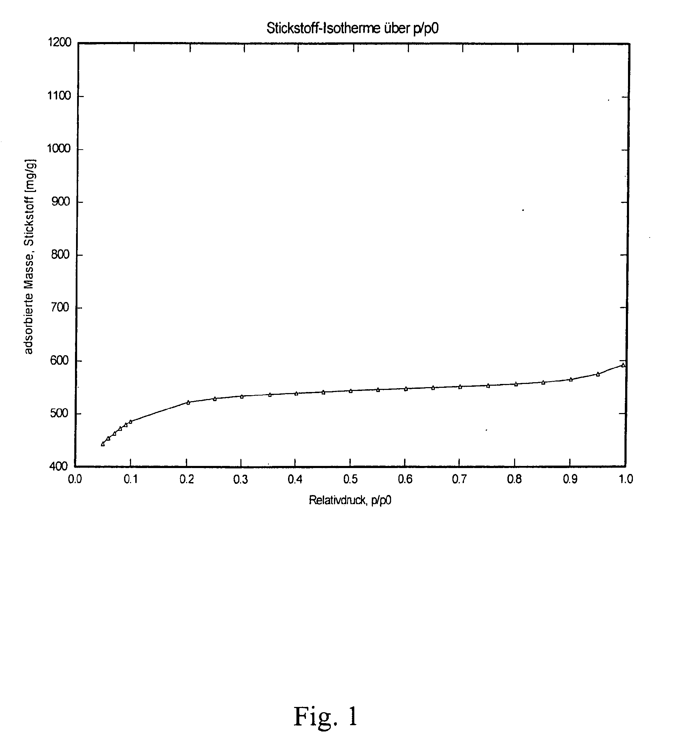

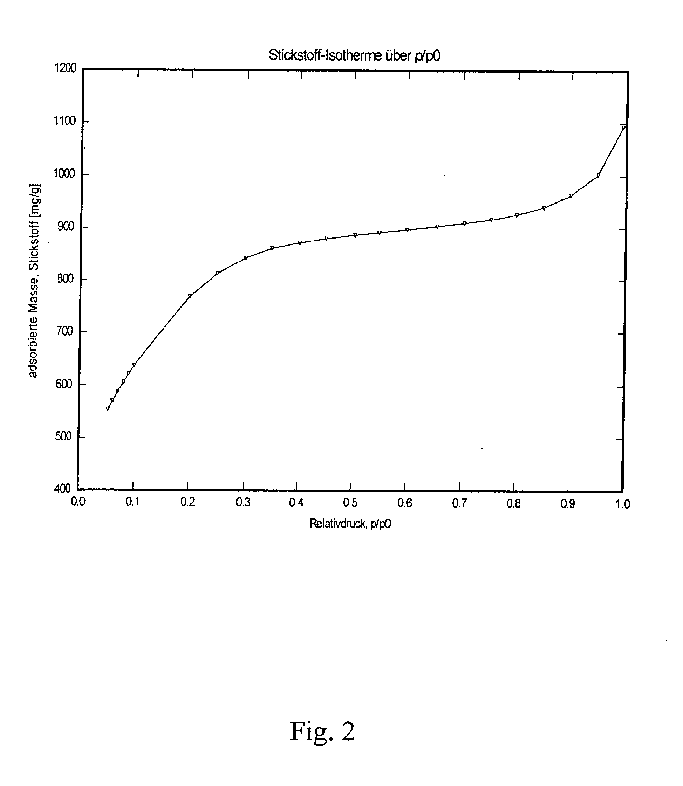

Die Figurendarstellungen gemäß Fig. 1 und Fig. 2 zeigen N₂-Adsorptionsisothermen für zwei unterschiedliche erfindungsgemäße Hochleistungsadsorbentien, welche mit unterschiedlich langen Aktivierungsdauern hergestellt worden sind. Die physikochemischen Eigenschaften der beiden erfindungsgemäßen Hochleistungsadsorbentien sind zudem in der nachfolgenden Tabelle 1 zusammengefaßt. Zum Vergleich ist dort auch eine handelsübliche mikroporöse Aktivkohle der Fa. Kureha mit den betreffenden physikochemischen Eigenschaften aufgeführt.

Die in Tabelle 1 wiedergegebenen Daten zeigen die Überlegenheit der erfindungsgemäßen Hochleistungsadsorbentien gegenüber einer mikroporösen Aktivkohle des Standes der Technik: Die Kombination einer hohen Gesamtporosität mit hohem Mikroporenvolumenanteil bei hoher BET-Oberfläche und gleichzeitig hoher mechanischer Beständigkeit (Druck- bzw. Berstfestigkeit sowie Abriebfestigkeit) und exzellenten Adsorptionseigenschaften (hohe Butanadsorption und Iodzahl) ist in dieser Kombination - neben den übrigen physikochemischen Parametern - nur bei der erfindungsgemäßen Hochleistungsadsorbentien zu finden. Erfindungsgemäß lassen sich also hochmikroporöse Hochleistungsadsorbentien auf Basis von Aktivkohle in Kornform, insbesondere Kugelform, herstellen, welche den handelsüblichen Produkten überlegen sind.

Die in Tabelle 1 aufgeführten erfindungsgemäßen Hochleistungsadsorbentien "Aktivkohle I" und "Aktivkohle II" werden jeweils wie folgt hergestellt: Handelsübliche Ionenaustauschervorstufen vom Geltyp auf der Basis von divinylbenzolvernetzten Polystyrolcopolymeren mit einem Divinylbenzolgehalt von etwa 4 % werden zunächst vorgetrocknet, um den etwa 50%igen Wasseranteil zu entfernen, und nachfolgend bei Temperaturen von 100 °C bis 150 °C mit einer Schwefelsäure/Oleum-Mischung in an sich bekannter Weise sulfoniert. Dann wird in an sich bekannter Weise bei Temperaturen bis zu 950 °C vier Stunden lang unter Stickstoffatmosphäre carbonisiert und anschließend die Aktivierung eingeleitet, indem der Stickstoffatmosphäre geringe Mengen an Wasserdampf (ca. 1 bis 3 Vol.-%) zugesetzt werden; die Wasserdampfzufuhr wird beibehalten, um den Wasserdampfanteil in dieser Weise zu regulieren. Die Aktivierung wird 19 Stunden lang ("Aktivkohle I") bzw. 23 Stunden lang ("Aktivkohle II") betrieben. Nach Abkühlung auf Raumtemperatur werden die in Tabelle 1 aufgeführten erfindungsgemäßen Produkte erhalten.

Weiterer Gegenstand der vorliegenden Erfindung - gemäß einem **zweiten** Aspekt der vorliegenden Erfindung - ist die erfindungsgemäße Verwendung der Hochleistungsadsorbentien nach der vorliegenden Erfindung.

So eignen sich die erfindungsgemäßen Hochleistungsadsorbentien insbesondere für die Adsorption von Giftstoffen, Schadstoffen und Gerüchen, beispielsweise aus Gas- bzw. Luftströmen. Weiterhin eignen sich die erfindungsgemäßen Hochleistungsadsorbentien auch zur Reinigung und Aufbereitung von Gasen, insbesondere zur Reinigung von Luft.

Weiterhin können die erfindungsgemäßen Hochleistungsadsorbentien auch in Adsorptionsfiltermaterialien bzw. zur Herstellung von Adsorptionsfiltermaterialien verwendet werden. Derartige Adsorptionsfiltermaterialien lassen sich insbesondere für die Herstellung von Schutzbekleidung einsetzen, z. B. von Schutzanzügen, Schutzhandschuhen, Schutzunterwäsche, Schutzschuhen etc., insbesondere für den zivilen oder militärischen Bereich (z. B. ABC-Schutz).

Des weiteren eignen sich die erfindungsgemäßen Hochleistungsadsorbentien auch zur Verwendung im Bereich der Medizin oder Pharmazie, insbesondere als Arzneimittel oder Arzneimittelbestandteil.

Schließlich können die erfindungsgemäßen Hochleistungsadsorbentien auch als Sorptionsspeicher für Gase, insbesondere Wasserstoff, dienen.

Aufgrund ihrer hohen Gesamtporosität bei hoher Mikroporosität und gleichzeitig exzellenter mechanischer Beständigkeit mit exzellenten adsorptiven Eigenschaften sind die erfindungsgemäßen Hochleistungsadsorbentien den Adsorbentien des Standes der Technik mit vergleichbarer Mikroporosität deutlich überlegen.

**Tabelle 1: Vergleich physikochemischer Parameter von zwei erfindungsgemäßen Hochleistungsadsorbentien auf der Basis kugelförmiger Aktivkohle einerseits und handelsüblicher mikroporöser Aktivkohle in Kugelform der Fa. Kureha andererseits**

| | Erfindungsgemäße Aktivkohle I | Erfindungsgemäße Aktivkohle II | Handelsübliche Aktivkohle der Fa. Kureha |
|---|---|---|---|
| Gesamtporenvolumen (Gurvich) (p/p₀ =0,995) [cm³/g] ** | 0,7336 | 1,3550 | 0,5891 |
| Mittlerer Porendurchmesser [Å] | 18,57 | 24,67 | 17,89 |
| BET (Multipoint, MP) (p/p₀ = 0,05-0,1) (ASTM D6556-04) [m²/g] ** | 1.580 | 2.197 | 1.317 |
| Mikroporenvolumen (Carbon Black) [cm³/g] * | 0,6276 | 0,9673 | 0,5240 |
| Anteil der Mikroporen am Gesamtporenvolumen [%] * | 85,55 | 71,39 | 88,95 |
| Adsorbiertes Volumen N₂ (p/p₀ = 0,25) gewichtsbezogen [cm³/g] ** | 423 | 650 | 349 |
| Adsorbiertes Volumen N₂ (p/p₀ = 0,25) volumenbezogen [cm³/ cm³]** | 236 | 235 | 206 |
| Adsorbiertes Volumen N₂ (p/p₀ = 0,995) gewichtsbezogen [cm³/g] ** | 473 | 770 | 380 |
| Adsorbiertes Volumen N₂ (p/p₀ = 0,995) volumenbezogen [cm³/ cm³]** | 264 | 279 | 224 |
| Mikroporenoberfläche (Carbon Black) [cm²/g] * | 1.509 | 1.995 | 1.271 |
| Äußeres Porenvolumen (Carbon Black) [cm³/g] | 0,11 | 0,39 | 0,07 |
| Anteil äußeres Porenvolumen in bezug auf Gesamtporenvolumen [%] | 14,4 | 28,6 | 11,1 |
| Äußere Porenoberfläche (Carbon Black) [cm²/g] | 71 | 202 | 46 |
| Anteil der äußeren Porenoberfläche in bezug auf BET-Oberfläche (MP) [%] | 4,5 | 9,2 | 3,5 |
| Adsorbat | N₂ | N₂ | N₂ |
| Butanadsorption (ASTM D5742-95/00) [%] | 33,5 | 59,6 | 29,2 |
| Iodzahl (ASTM D4607-94/99) [mg/g] | 1.470 | 1.840 | 1.343 |
| Druck- bzw. Berstfestigkeit (Gewichtsbelastbarkeit) [kg/Aktivkohlekügelchen] | 3,75 | 1,4 | 0,45 |
| Mittlerer Durchmesser (ASTM D2862-97/04) [mm] | 0,52 | 0,44 | 0,44 |
| Aschegehalt (ASTM D2866-94/04) [%] | 0,50 | 0,45 | 0,04 |
| Feuchtigkeitsgehalt (ASTM D2867-04/04) [%] | 0,04 | 0,1 | 0,37 |

| | | | |
|---|---|---|---|
| * Mikroporen: Poren mit Porendurchmessern ≤ 20 Å bezeichnet **p/p₀ = Partialdruck oder Partialdruckbereich | | | |

## Patentansprüche

1. Hochleistungsadsorbentien auf der Basis von Aktivkohle in Kugelform mit mittleren Teilchendurchmessern, bestimmt nach ASTM D2862-97/04, im Bereich von 0,01 bis 1,0 mm,
**gekennzeichnet**
**durch** die folgenden Parameter:
• ein Gesamtporenvolumen nach Gurvich von mindestens 0,7 cm³/g, wobei mindestens 70 % dieses Gesamtporenvolumens durch Mikroporen mit Porendurchmessern von ≤ 2 nm (20 Å) gebildet sind,
• einen mittleren Porendurchmesser von höchstens 3 nm (30 Å), bestimmt auf Basis der Stickstoffisothermen,
• eine BET-Oberfläche von mindestens 1.500 m²/g, bestimmt nach ASTM D6556-04, und
• eine Schüttdichte, bestimmt nach ASTM B527-93/00, im Bereich von 350 bis 600 g/l.

2. Hochleistungsadsorbentien auf der Basis von Aktivkohle nach Anspruch 1, **dadurch gekennzeichnet, dass** das Gesamtporenvolumen nach Gurvich der Hochleistungsadsorbentien mindestens 0,8 cm³/g, insbesondere mindestens 0,9 cm³/g, vorzugsweise mindestens 1,0 cm³/g, beträgt und/oder Werte bis zu 1,5 cm³/g, insbesondere bis zu 1,6 cm³/g, vorzugsweise bis zu 1,8 cm³/g, erreicht und/oder das Gesamtporenvolumen nach Gurvich der Hochleistungsadsorbentien im Bereich von 0,7 bis 1,8 cm³/g, insbesondere 0,8 bis 1,6 cm³/g, vorzugsweise 0,9 bis 1,5 cm³/g, liegt.

3. Hochleistungsadsorbentien auf der Basis von Aktivkohle nach Anspruch 1 oder 2, **dadurch gekennzeichnet, dass** mindestens 75 %, insbesondere mindestens 80 %, vorzugsweise mindestens 85 %, besonders bevorzugt mindestens 90 %, des Gesamtporenvolumens nach Gurvich der Hochleistungsadsorbentien durch Mikroporen mit Porendurchmessern von ≤ 2 nm (20 Å) gebildet sind und/oder dass 70 % bis 95 %, insbesondere 75 % bis 90 %, vorzugsweise 75 % bis 85 %, des Gesamtporenvolumens nach Gurvich der Hochleistungsadsorbentien durch Mikroporen mit Porendurchmessern von ≤ 2 nm (20 Å) gebildet sind.

4. Hochleistungsadsorbentien auf der Basis von Aktivkohle nach einem oder mehreren der vorangehenden Ansprüche, **dadurch gekennzeichnet, dass** das durch Mikroporen mit Porendurchmessern von ≤ 2 nm (20 Å) gebildete Mikroporenvolumen nach Carbon Black der Hochleistungsadsorbentien im Bereich von 0,5 bis 1,4 cm³/g, insbesondere 0,6 bis 1,2 cm³/g, bevorzugt 0,7 bis 1,1 cm³/g, liegt.

5. Hochleistungsadsorbentien auf der Basis von Aktivkohle nach einem oder mehreren der vorangehenden Ansprüche, **dadurch gekennzeichnet, dass** der mittlere Porendurchmesser der Hochleistungsadsorbentien höchstens 2,6 nm (26 Å), insbesondere höchstens 2,5 nm (25 Å), bevorzugt höchstens 2,4 nm (24 Å), beträgt und/oder dass der mittlere Porendurchmesser der Hochleistungsadsorbentien im Bereich von 1,5 bis 3 nm (15 bis 30 Å), insbesondere 1,6 bis 2,6 nm (16 bis 26 Å), vorzugsweise 1,7 bis 2,5 nm (17 bis 25 Å), besonders bevorzugt 1,8 bis 2,4 nm (18 bis 24 Å), liegt.

6. Hochleistungsadsorbentien auf der Basis von Aktivkohle nach einem oder mehreren der vorangehenden Ansprüche, **dadurch gekennzeichnet, dass** die BET-Oberfläche der Hochleistungsadsorbentien im Bereich von 1.500 m²/g bis 2.750 m²/g, insbesondere 1.525 bis 2.500 m²/g, vorzugsweise 1.550 bis 2.400 m²/g, besonders bevorzugt 1.575 bis 2.350 m²/g, liegt.

7. Hochleistungsadsorbentien auf der Basis von Aktivkohle nach einem oder mehreren der vorangehenden Ansprüche, **dadurch gekennzeichnet, dass** das gewichtsbezogene adsorbierte N₂-Volumen V_{ads (gew.)} der Hochleistungsadsorbentien, bestimmt bei einem Partialdruck p/p₀ von 0,25, mindestens 400 cm³/g, insbesondere mindestens 420 cm³/g, beträgt und insbesondere im Bereich von 400 bis 800 cm³/g, vorzugsweise 410 bis 750 cm³/g, besonders bevorzugt 420 bis 700 cm³/g, liegt und/oder dass das volumenbezogene adsorbierte N₂-Volumen V_{ads(vol.)} der Hochleistungsadsorbentien, bestimmt bei einem Partialdruck p/p₀ von 0,25, mindestens 200 cm³/cm³, insbesondere mindestens 220 cm³/cm³, beträgt und insbesondere im Bereich von 200 bis 300 cm³/cm³, vorzugsweise 210 bis 275 cm³/cm³, besonders bevorzugt 225 bis 260 cm³/cm³, liegt.

8. Hochleistungsadsorbentien auf der Basis von Aktivkohle nach einem oder mehreren der vorangehenden Ansprüche, **dadurch gekennzeichnet, dass** das gewichtsbezogene adsorbierte N₂-Volumen V_{ads (gew.)} der Hochleistungsadsorbentien, bestimmt bei einem Partialdruck p/p₀ von 0,995, mindestens 450 cm³/g, insbesondere mindestens 460 cm³/g, beträgt und insbesondere im Bereich von 450 bis 900 cm³/g, vorzugsweise 460 bis 875 cm³/g, besonders bevorzugt 470 bis 850 cm³/g, liegt und/oder dass das volumenbezogene adsorbierte N₂-Volumen V_{ads (vol.)} der Hochleistungsadsorbentien, bestimmt bei einem Partialdruck p/p₀ von 0,995, mindestens 250 cm³/cm³, insbesondere mindestens 260 cm³/cm³, beträgt und insbesondere im Bereich von 250 bis 400 cm³/cm³, vorzugsweise 260 bis 350 cm³/cm³, besonders bevorzugt 265 bis 320 cm³/cm³, liegt.

9. Hochleistungsadsorbentien auf der Basis von Aktivkohle nach einem oder mehreren der vorangehenden Ansprüche, **dadurch gekennzeichnet, dass** die aus Poren mit Porendurchmessern von ≤ 2 nm (20 Å) gebildete Mikroporenoberfläche nach Carbon Black der Hochleistungsadsorbentien mindestens 1.400 m²/g, insbesondere mindestens 1.450 m²/g, vorzugsweise mindestens 1.500 m²/g, beträgt und/oder dass die aus Poren mit Porendurchmessern von ≤ 2 nm (20 Å) gebildete Mikroporenoberfläche nach Carbon Black der Hochleistungsadsorbentien im Bereich von 1.400 bis 2.500 m²/g, insbesondere 1.450 bis 2.400 m²/g, vorzugsweise 1.500 bis 2.300 m²/g, beträgt.

10. Hochleistungsadsorbentien auf der Basis von Aktivkohle nach einem oder mehreren der vorangehenden Ansprüche, **dadurch gekennzeichnet, dass** die Hochleistungsadsorbentien eine Butanadsorption von mindestens 25 %, insbesondere mindestens 30 %, vorzugsweise mindestens 40 %, aufweisen und/oder dass die Hochleistungsadsorbentien eine Butanadsorption im Bereich von 25 bis 80 %, insbesondere 30 bis 70 %, vorzugsweise 35 bis 65 %, aufweisen und/oder dass die Hochleistungsadsorbentien eine Iodzahl von mindestens 1.350 mg/g, insbesondere mindestens 1.450 mg/g, bevorzugt mindestens 1.500 mg/g, aufweisen und/oder dass die Hochleistungsadsorbentien eine Iodzahl im Bereich von 1.350 bis 2.100 mg/g, insbesondere 1.450 bis 2.050 mg/g, vorzugsweise 1.500 bis 2.000 mg/g, aufweisen.

11. Hochleistungsadsorbentien auf der Basis von Aktivkohle nach einem oder mehreren der vorangehenden Ansprüche, **dadurch gekennzeichnet, dass** das äußere Porenvolumen nach Carbon Black der Hochleistungsadsorbentien im Bereich von 0,05 bis 0,5 cm³/g, insbesondere 0,1 bis 0,45 cm³/g, liegt und/oder dass das äußere Porenvolumen nach Carbon Black der Hochleistungsadsorbentien höchstens 35 %, vorzugsweise höchstens 30 %, des Gesamtporenvolumens und insbesondere 10 % bis 35 %, bevorzugt 14 bis 30 %, des Gesamtporenvolumens bildet.

12. Hochleistungsadsorbentien auf der Basis von Aktivkohle nach einem oder mehreren der vorangehenden Ansprüche, **dadurch gekennzeichnet, dass** die äußere Porenoberfläche nach Carbon Black der Hochleistungsadsorbentien im Bereich von 50 bis 300 m²/g, insbesondere 60 bis 250 m²/g, bevorzugt 70 bis 200 m²/g, liegt und/oder dass die äußere Porenoberfläche nach Carbon Black der Hochleistungsadsorbentien höchstens 15 %, vorzugsweise höchstens 10 %, der Gesamtporenoberfläche und insbesondere 4 bis 15 %, bevorzugt 4 bis 12 %, der Gesamtporenoberfläche bildet.

13. Hochleistungsadsorbentien auf der Basis von Aktivkohle in Kugelform nach einem oder mehreren der vorangehenden Ansprüche, **gekennzeichnet durch** die folgenden Parameter:
• ein Gesamtporenvolumen nach Gurvich von mindestens 0,7 cm³/g, wobei mindestens 70 % dieses Gesamtporenvolumens **durch** Mikroporenporen mit Porendurchmessern von ≤ 2 nm (20 Å) gebildet sind,
• einen mittleren Porendurchmesser von höchstens 3 nm (30 Å), bestimmt auf Basis der Stickstoffisothermen,
• eine BET-Oberfläche von mindestens 1.500 m²/g, bestimmt nach ASTM D6556-04,
• eine Butanadsorption von mindestens 25 %, bestimmt nach ASTM D5742-95/00,
• eine Iodzahl von mindestens 1.350 mg/g, bestimmt nach ASTM D4607-94/99,
• eine Schüttdichte, bestimmt nach ASTM B527-93/00, im Bereich von 350 bis 600 g/l und
• gegebenenfalls eine Druck- bzw. Berstfestigkeit pro Aktivkohlekorn von mindestens 10 Newton und/oder gegebenenfalls eine Abriebfestigkeit von mindestens 95 %.

14. Verwendung der Hochleistungsadsorbentien auf der Basis von Aktivkohle nach einem oder mehreren der vorangehenden Ansprüche für die Adsorption von Giftstoffen, Schadstoffen und Gerüchen, insbesondere aus Gas- oder Luftströmen, oder zur Reinigung oder Aufbereitung von Gasen, insbesondere Luft.

15. Verwendung der Hochleistungsadsorbentien auf der Basis von Aktivkohle nach einem oder mehreren der Ansprüche 1 bis 13 zur Verwendung in Adsorptionsfiltermaterialien, insbesondere für die Herstellung von Schutzbekleidung.

16. Verwendung der Hochleistungsadsorbentien auf der Basis von Aktivkohle nach einem oder mehreren der Ansprüche 1 bis 13 als Sorptionsspeicher für Gase, insbesondere Wasserstoff.

17. Verwendung der Hochleistungsadsorbentien auf der Basis von Aktivkohle nach einem oder mehreren der Ansprüche 1 bis 13 im Bereich der Medizin oder Pharmazie, insbesondere als Arzneimittel oder Arzneimittelbestandteil.

## Claims

1. High-performance absorbents based on activated carbon in spherical form, having mean particle diameters in the range of 0.01 to 1.0 mm, as determined by ASTM D2862-97/04,
**characterized by**
the following parameters:
• a total pore volume according to Gurvich of at least 0.7 cm³/g, wherein at least 70% of said total pore volume is formed by micropores, having pore diameters of ≤ 2 nm (20 Å),
• a mean pore diameter of not more than 3 nm (30 Å),
as determined based on the nitrogen isotherms
• a BET surface of at least 1,500 m²/g, as determined by ASTM D6556-04, and
• a bulk density in the range of 350 to 600 g/l, as determined by ASTM B527-93/00.

2. The high-performance absorbents based on activated carbon according to claim 1, **characterized in that** the total pore volume according to Gurvich of the high-performance absorbents is at least 0.8 cm³/g, particularly at least 0.9 cm³/g, preferably at least 1.0 cm³/g, and/or reaches values of up to 1.5 cm³/g, particularly up to 1.6 cm³/g, preferably up to 1.8 cm³/g, and/or the total pore volume according to Gurvich of the high-performance adsorbents is in the range of 0.7 to 1.8 cm³/g, particularly 0.8 to 1.6 cm³/g, preferably 0.9 to 1.5 cm³/g.

3. The high-performance absorbents based on activated carbon according to claims 1 or 2, **characterized in that** at least 75%, particularly at least 80%, preferably at least 85%, particularly preferred at least 90% of the total pore volume according to Gurvich of the high-performance adsorbents are formed by micropores having pore diameters of ≤ 2 nm (20 Å), and/or that 70% to 95%, particularly 75% to 90%, preferably 75% to 85% of the total pore volume according to Gurvich of the high-performance adsorbents are formed by micropores having pore diameters of ≤ 2 nm (20 Å).

4. The high-performance absorbents based on activated carbon according to one or more of the previous claims, **characterized in that** the micropore volume according to carbon black of the high-performance adsorbents formed by micropores having a pore diameter of ≤ 2 nm (20 Å) is in the range of 0.5 to 1.4 cm³/g, particularly 0.6 to 1.2 cm³/g, preferably 0.7 to 1.1 cm³/g.

5. The high-performance absorbents based on activated carbon according to one or more of the previous claims, **characterized in that** the mean pore diameter of the high-performance adsorbents is not more than 2.6 nm (26 Å), preferably not more than 2.4 nm (24 Å), and/or that the mean pore diameter of the high-performance adsorbents is in the range of 1.5 to 3 nm (15 to 30 Å), particularly 1.6 to 2.6 (16 to 26 Å), preferably 1.7 to 2.5 nm (17 to 25 Å), particularly preferred 1.8 to 2.4 nm (18 to 24 Å).

6. The high-performance absorbents based on activated carbon according to one or more of the previous claims, **characterized in that** the BET surface of the high-performance adsorbents is in the range of 1,500 m²/g to 2,750 m²/g, particularly 1,525 to 2,500 m²/g, preferably 1,550 to 2,400 m²/g, particularly preferred 1,575 to 2,350 m²/g.

7. The high-performance absorbents based on activated carbon according to one or more of the previous claims, **characterized in that** the weight-based adsorbed N₂ volume V_{ads(weight)} of the high-performance absorbents, as determined at a partial pressure p/p₀ of 0.25, is at least 400 cm³/g, particularly at least 420 cm³/g, and in particular in the range of 400 to 800 cm³/g, preferably 410 to 750 cm³/g, particularly preferred 420 to 700 cm³/g, and/or that the volume-based adsorbed N₂ volume V_{ads(vol.)} of the high-performance absorbents, as determined at a partial pressure p/p₀ of 0.25, is at least 200 cm³/cm³, particularly at least 220 cm³/cm³, and in particular in the range of 200 to 300 cm³/cm³, preferably 210 to 275 cm³/cm³, particularly preferred 225 to 260 cm³/cm³.

8. The high-performance absorbents based on activated carbon according to one or more of the previous claims, **characterized in that** the weight-based adsorbed N₂ volume V_{ads(weight)} of the high-performance absorbents, as determined at a partial pressure p/p₀ of 0.995, is at least 450 cm³/g, particularly at least 460 cm³/g, and particularly in the range of 450 to 900 cm³/g, preferably 460 to 875 cm³/g, particularly preferred 470 to 850 cm³/g, and/or that the volume-based adsorbed N₂ volume V_{ads(vol.)} of the high-performance absorbents, as determined at a partial pressure p/p₀ of 0.995, is at least 250 cm³/cm³, particularly at least 260 cm³/cm³, and in particular in the range of 250 to 400 cm³/cm³, preferably 260 to 350 cm³/cm³, particularly preferred 265 to 320 cm³/cm³.

9. The high-performance absorbents based on activated carbon according to one or more of the previous claims, **characterized in that** the micropore surface according to carbon black of the high-performance absorbents formed from pores having pore diameters of ≤ 2 nm (20 Å) is at least 1,400 m²/g, particularly at least 1,450 m²/g, preferably at least 1,500 m²/g, and/or that the micropore surface according to carbon black of the high-performance absorbents formed from pores having pore diameters of ≤ 2 nm (20 Å) is in the range of 1,400 to 2,500 m²/g, particularly 1,450 to 2,400 m²/g, preferably 1,500 to 2,300 m²/g.

10. The high-performance absorbents based on activated carbon according to one or more of the previous claims, **characterized in that** the high-performance absorbents have a butane adsorption of at least 25%, in particular at least 30%, preferably at least 40%, and/or that the high-performance absorbents have a butane adsorption in the range of 25 to 80%, particularly 30 to 70%, preferably 35 to 65%, and/or that the high-performance absorbents have an iodine count of at least 1,350 mg/g, particularly at least 1,450 mg/g, preferably at least 1,500 mg/g, and/or that the high-performance absorbents have an iodine count in the range of 1,350 to 2,100 mg/g, particularly 1,450 to 2,050 mg/g, preferably 1,500 to 2,000 mg/g.

11. The high-performance absorbents based on activated carbon according to one or more of the previous claims, **characterized in that** the outer pore volume according to carbon black of the high-performance absorbents is in the range of 0.05 to 0.5 cm³/g, particularly 0.1 to 0.45 cm³/g, and/or that the outer pore volume according to carbon black of the high-performance absorbents is not more than 35%, preferably not more than 30% of the total pore volume, and in particular forms 10% to 35%, preferably 14 to 30% of the total pore volume.

12. The high-performance absorbents based on activated carbon according to one or more of the previous claims, **characterized in that** the outer pore surface according to carbon black of the high-performance absorbents is in the range of 50 to 300 m²/g, particularly 60 to 250 m²/g, preferably 70 to 200 m²/g, and/or that the outer pore surface according to carbon black of the high-performance absorbents forms not more than 15%, preferably 10% of the total pore surface, and in particular forms 4 to 15%, preferably 4 to 12% of the total pore surface.

13. The high-performance absorbents based on activated carbon in spherical form according to one or more of the previous claims, **characterized by** the following parameters:
• a total pore volume according to Gurvich of at least 0.7 cm³/g, wherein at least 70% of said total pore volume is formed by micropores, having pore diameters of ≤ 2 nm (20 Å),
• a mean pore diameter of not more than 3 nm (30 Å), as determined based on the nitrogen isotherms,
• a BET surface of at least 1,500 m²/g, as determined by ASTM D6556-04,
• a butane adsorption of at least 25%, as determined by ASTM D5742-95/00
• an iodine count of at least 1,350 mg/g, as determined by ASTM D4607-94/99
• a bulk density in the range of 350 to 600 g/I, as determined by ASTM B527-93/00, and
• optionally, a compression or bursting strength per active carbon core of at least 10 Newtons, and/or optionally an abrasion resistance of at least 95%.

14. A use of the high-performance absorbents based on activated carbon according to one or more of the previous claims for the adsorption of toxins, contaminants, and odors, in particular from gas or air streams, or for purifying or processing gases, in particular air.

15. The use of the high-performance absorbents based on activated carbon according to one or more of claims 1 to 13 for use in adsorption filter materials, in particular for the production of protective clothing.

16. The use of the high-performance absorbents based on activated carbon according to one or more of claims 1 to 13 as sorption storage for gases, in particular hydrogen.

17. The use of the high-performance absorbents based on activated carbon according to one or more of claims 1 to 13 within the scope of medicine or pharmaceuticals, in particular as a pharmaceutical, or a component of a pharmaceutical.

## Revendications

1. Adsorbants hautes performances à base de charbon actif sous forme sphérique ayant une granulométrie moyenne, déterminée selon ASTM D2862-97/04, comprise dans la plage de 0,01 à 1,0 mm,
**caractérisés par** les paramètres suivants :
- un volume total des pores selon Gurvich d'au moins 0,7 cm³/g, au moins 70 % de ce volume total des pores étant formés de micropores ayant un diamètre de pore ≤ 2 nm (20 Å),
- un diamètre moyen des pores d'au plus 3 nm (30 Å), déterminé sur la base des isothermes de l'azote,
- une aire BET d'au moins 1500 m²/g, déterminée selon ASTM D6556-04, et
- une masse volumique apparente, déterminée selon ASTM B527-93/00, comprise dans la plage de 350 à 600 g/l.

2. Adsorbants hautes performances à base de charbon actif selon la revendication 1, **caractérisés en ce que** le volume total des pores selon Gurvich des adsorbants hautes performances est d'au moins 0,8 cm³/g, en particulier d'au moins 0,9 cm³/g, de préférence d'au moins 1,0 cm³/g, et/ou atteint des valeurs allant jusqu'à 1,5 cm³/g, en particulier jusqu'à 1,6 cm³/g, de préférence jusqu'à 1,8 cm³/g, et/ou que le volume total des pores selon Gurvich des adsorbants hautes performances est compris dans la plage de 0,7 à 1,8 cm³/g, en particulier de 0,8 à 1,6 cm³/g, de préférence de 0,9 à 1,5 cm³/g.

3. Adsorbants hautes performances à base de charbon actif selon la revendication 1 ou 2, **caractérisés en ce qu'**au moins 75 %, en particulier au moins 80 %, de préférence au moins 85 %, d'une manière particulièrement préférée au moins 90 % du volume total des pores selon Gurvich des adsorbants hautes performances sont formés de micropores ayant un diamètre de pore ≤ 2 nm (20 Å), et/ou que 70 % à 95 %, en particulier 75 % à 90 %, de préférence 75 % à 85 % du volume total des pores selon Gurvich des adsorbants hautes performances sont formés de micropores ayant un diamètre de pore ≤ 2 nm (20 Å).

4. Adsorbants hautes performances à base de charbon actif selon l'une ou plusieurs des revendications précédentes, **caractérisés en ce que** le volume des micropores formé de micropores ayant un diamètre de pore ≤ 2 nm (20 Å), déterminé par la méthode au noir de carbone, des adsorbants hautes performances est compris dans la plage de 0,5 à 1,4 cm³/g, en particulier de 0,6 à 1,2 cm³/g, de préférence de 0,7 à 1,1 cm³/g.

5. Adsorbants hautes performances à base de charbon actif selon l'une ou plusieurs des revendications précédentes, **caractérisés en ce que** le diamètre moyen des pores des adsorbants hautes performances est d'au plus 2,6 nm (26 Å), en particulier d'au plus 2,5 nm (25 Å), de préférence d'au plus 2,4 nm (24 Å), et/ou que le diamètre moyen des pores des adsorbants hautes performances est compris dans la plage de 1,5 à 3 nm (15 à 30 Å), en particulier de 1,6 à 2,6 nm (16 à 26 Å), de préférence de 1,7 à 2,5 nm (17 à 25 Å), d'une manière particulièrement préférée de 1,8 à 2,4 nm (18 à 24 Å).

6. Adsorbants hautes performances à base de charbon actif selon l'une ou plusieurs des revendications précédentes, **caractérisés en ce que** l'aire BET des adsorbants hautes performances est comprise dans la plage de 1500 m²/g à 2750 m²/g, en particulier de 1525 à 2500 m²/g, de préférence de 1550 à 2400 m²/g, d'une manière particulièrement préférée de 1575 à 2350 m²/g.

7. Adsorbants hautes performances à base de charbon actif selon l'une ou plusieurs des revendications précédentes, **caractérisés en ce que** le volume de N₂ adsorbé V_{ads(gew.)}, rapporté au poids, des adsorbants hautes performances, déterminé sous une pression partielle p/p₀ de 0,25, est d'au moins 400 cm³/g, en particulier d'au moins 420 cm³/g, et est en particulier compris dans la plage de 400 à 800 cm³/g, de préférence de 410 à 750 cm³/g, d'une manière particulièrement préférée de 420 à 700 cm³/g, et/ou que le volume de N₂ adsorbé V_{ads(vol.)}, rapporté au volume, des adsorbants hautes performances, déterminé sous une pression partielle p/p₀ de 0,25, est d'au moins 200 cm³/cm³, en particulier d'au moins 220 cm³/cm³, et en particulier est compris dans la plage de 200 à 300 cm³/cm³, de préférence de 210 à 275 cm³/cm³, d'une manière particulièrement préférée de 225 à 260 cm³/cm³.

8. Adsorbants hautes performances à base de charbon actif selon à base de charbon actif selon l'une ou plusieurs des revendications précédentes, **caractérisés en ce que** le volume de N₂ adsorbé V_{ads(gew.)}, rapporté au poids, des adsorbants hautes performances, déterminé sous une pression partielle p/p₀ de 0,995, est d'au moins 450 cm³/g, en particulier d'au moins 460 cm³/g, et est en particulier compris dans la plage de 450 à 900 cm³/g, de préférence de 460 à 875 cm³/g, d'une manière particulièrement préférée de 470 à 850 cm³/g, et/ou que le volume de N₂ adsorbé V_{ads(vol.)}, rapporté au volume, des adsorbants hautes performances, déterminé sous une pression partielle p/p₀ de 0,995, est d'au moins 250 cm³/cm³, en particulier d'au moins 260 cm³/cm³, et est en particulier compris dans la plage de 250 à 400 cm³/cm³, de préférence de 260 à 350 cm³/cm³, d'une manière particulièrement préférée de 265 à 320 cm³/cm³.

9. Adsorbants hautes performances à base de charbon actif selon l'une ou plusieurs des revendications précédentes, **caractérisés en ce que** l'aire des micropores formés de pores ayant un diamètre de pore ≤ 2 nm (20 Å), déterminée par la méthode au noir de carbone, des adsorbants hautes performances est d'au moins 1400 m²/g, en particulier d'au moins 1450 m²/g, de préférence d'au moins 1500 m²/g, et/ou que l'aire des micropores formés de pores ayant un diamètre de pore ≤ 2 nm (20 Å), déterminée par la méthode au noir de carbone, des adsorbants hautes performances est comprise dans la plage de 1400 à 2500 m²/g, en particulier de 1450 à 2400 m²/g, de préférence de 1500 à 2300 m²/g.

10. Adsorbants hautes performances à base de charbon actif selon l'une ou plusieurs des revendications précédentes, **caractérisés en ce que** les adsorbants hautes performances présentent une adsorption du butane d'au moins 25 %, en particulier d'au moins 30 %, de préférence d'au moins 40 %, et/ou que les adsorbants hautes performances présentent une adsorption du butane comprise dans la plage de 25 à 80 %, en particulier de 30 à 70 %, de préférence de 35 à 65 %, et/ou que les adsorbants hautes performances présentent un indice d'iode d'au moins 1350 mg/g, en particulier d'au moins 1450 mg/g, de préférence d'au moins 1500 mg/g, et/ou que les adsorbants hautes performances présentent un indice d'iode compris dans la plage de 1350 à 2100 mg/g, en particulier de 1450 à 2050 mg/g, de préférence de 1500 à 2000 mg/g.

11. Adsorbants hautes performances à base de charbon actif selon l'une ou plusieurs des revendications précédentes, **caractérisés en ce que** le volume extérieur des pores, déterminé par la méthode au noir de carbone, des adsorbants hautes performances, est compris dans la plage de 0,05 à 0,5 cm³/g, en particulier de 0,1 à 0,45 cm³/g, et/ou que le volume extérieur des pores, déterminé par la méthode au noir de carbone, des adsorbants hautes performances représente au plus 35 %, de préférence au plus 30 % du volume total des pores, et en particulier 10 % à 35 %, de préférence 14 à 30 % du volume total des pores.

12. Adsorbants hautes performances à base de charbon actif selon l'une ou plusieurs des revendications précédentes, **caractérisés en ce que** l'aire extérieure des pores, déterminée par la méthode au noir de carbone, des adsorbants hautes performances est comprise dans la plage de 50 à 300 m²/g, en particulier de 60 à 250 m²/g, de préférence de 70 à 200 m²/g, et/ou que l'aire extérieure des pores, déterminée par la méthode au noir de carbone, des adsorbants hautes performances, représente au plus 15 %, de préférence au plus 10 % de l'aire totale des pores et en particulier 4 à 15 %, de préférence 4 à 12 % de l'aire totale des pores.

13. Adsorbants hautes performances à base de charbon actif sous forme sphérique selon l'une ou plusieurs des revendications précédentes, **caractérisés par** les paramètres suivants :
- un volume total des pores selon Gurvich d'au moins 0,7 cm³/g, au moins 70 % de ce volume total des pores étant formés de micropores ayant un diamètre de pore ≤ 2 nm (20 Å),
- un diamètre moyen des pores d'au plus 3 nm (30 Å), déterminé sur la base des isothermes de l'azote,
- une aire BET d'au moins 1500 m²/g, déterminée selon ASTM D6556-04,
- une adsorption du butane d'au moins 25 %, déterminée selon ASTM D5742-95/00
- un indice d'iode d'au moins 1350 mg/g, déterminé selon ASTM D4607-94/99,
- une masse volumique apparente, déterminée selon ASTM 8527-93/00, comprise dans la plage de 350 à 600 g/l, et
- éventuellement une résistance à la compression et à l'éclatement, par grain de charbon actif, d'au moins 10 Newton, et/ou éventuellement une résistance à l'abrasion d'au moins 95 %.

14. Utilisation des adsorbants hautes performances à base de charbon actif selon l'une ou plusieurs des revendications précédentes pour l'adsorption de substances toxiques, de substances nocives et d'odeurs, en particulier à partir de flux de gaz ou d'air, ou pour la purification ou la préparation de gaz, en particulier l'air.

15. Utilisation des adsorbants hautes performances à base de charbon actif selon l'une ou plusieurs des revendications 1 à 13, pour une utilisation dans des matériaux filtrants par adsorption, en particulier pour la fabrication de vêtements de protection.

16. Utilisation des adsorbants hautes performances à base de charbon actif selon l'une ou plusieurs des revendications 1 à 13 en tant qu'accumulateurs de sorption pour gaz, en particulier pour l'hydrogène.

17. Utilisation des adsorbants hautes performances à base de charbon actif selon l'une ou plusieurs des revendications 1 à 13 dans le domaine de la médecine ou de la pharmacie, en particulier en tant que médicament ou constituant de médicament.
